# EUROPEAN PATENT APPLICATION

(11) **EP 4 715 046 A1**
(43) Date of publication of application: **25.03.2026**
(21) Application number: 24807605.1
(22) Date of filing: 17.05.2024
(51) Int. Cl.: C12N 9/18, C12P 7/44, C08J 11/10

(54) **NOVEL PET DEGRADING ENZYME AND USE THEREOF**

(30) Priority: 18.05.2023 KR 20230064345
(71) Applicant: Zyen Co., Ltd., Daegu 41566 (KR); CJ Cheiljedang Corporation, Seoul 04560 (KR)
(72) Inventor: KIM, Kyung-Jin, Daegu 42013 (KR); JANG, Jaewon, Seoul 04560 (KR); HONG, Hwaseok, Seoul 04560 (KR); KIM, Hyung Joon, Seoul 04560 (KR)
(74) Representative: Meissner Bolte Partnerschaft mbB
(86) International application number: PCT/KR2024/006749
(87) International publication number: WO 2024/237734

(57) **Abstract**

Provided are a novel enzyme for degrading PET and use thereof.

## Description

### [Technical Field]

The present disclosure relates to a modified polypeptide having a PET degradation activity and use thereof.

### [Background Art]

Over 400 million tons of plastics are newly produced every year, and as environmental issues regarding waste plastics emerge, efforts are being made to reduce production thereof through regulations on single-use products and use of plastic substitutes. However, the use of plastics is actually increasing every year. PET, which accounts for less than 10% of all plastics, is newly produced at about 360 million tons every year, and is considered as a plastic with the shortest life cycle because it is mainly used for single-use products. Recycling of waste plastics comprises mechanical recycling, pyrolysis, chemical recycling, etc., and each method is commercialized or entering the final research stage for commercialization. Each technology may be a solution to the waste plastic issue, but no existing method can be considered perfect due to issues such as quality deterioration by downcycling, carbon neutrality, resource depletion, the impact on eutrophication of seawater and freshwater, etc.

In order to solve environmental problems caused by waste plastics, such as microplastics, greenhouse gas emissions, and resource depletion, etc., a series of research findings are being published for degrading PET, a representative type of plastic, by using a biological technology that utilizes enzymes (US Patent No. 10851355).

### [Disclosure]

### [Technical Problem]

The present disclosure relates to a novel polypeptide having a polyethylene terephthalate (PET) degradation activity, a method of degrading a polyester such as PET, etc. using the same, and a method of synthesizing a polyester such as PET, etc. by recycling mono(2-hydroxymethyl) terephthalate (MHET), terephthalic acid (TPA), and/or ethylene glycol (EG) which are obtained through the degradation.

### [Technical Solution]

An object of the present disclosure is to provide a modified polypeptide having a PET degradation activity.

Another object of the present disclosure is to provide a composition comprising the modified polypeptide.

Still another object of the present disclosure is to provide a polynucleotide encoding the modified polypeptide.

Still another object of the present disclosure is to provide a host cell comprising the modified polypeptide; the polynucleotide encoding the polypeptide; a nucleic acid construct comprising the polynucleotide; and/or a vector comprising the nucleotide or the nucleic acid construct.

Still another object of the present disclosure is to provide a method of preparing the modified polypeptide having the PET degradation activity.

Still another object of the present disclosure is to provide a method of degrading a polyester, the method comprising treating the polyester with the modified polypeptide; the host cell expressing the polypeptide; and/or the composition comprising the modified polypeptide.

Still another object of the present disclosure is to provide a method of producing mono(2-hydroxymethyl) terephthalate (MHET), terephthalic acid (TPA), and/or ethylene glycol (EG), the method comprising bringing a polyester into contact with the modified polypeptide; the host cell expressing the polypeptide; and/or the composition comprising the polypeptide.

Still another object of the present disclosure is to provide a method of producing a polyester, the method comprising the step of synthesizing the polyester using MHET, TPA, and/or EG produced by the above method.

Still another object of the present disclosure is to provide use of the modified polypeptide, the host cell expressing the modified polypeptide, and/or the composition comprising the modified polypeptide in degrading PET.

Still another object of the present disclosure is to provide use of the modified polypeptide, the host cell expressing the polypeptide, or the composition comprising the modified polypeptide for the reaction with a polyester in producing mono(2-hydroxymethyl) terephthalate (MHET), terephthalic acid (TPA), and/or ethylene glycol (EG).

### [Advantageous Effects]

By enzymatically degrading polyester bonds using a polypeptide having a PET degradation activity of the present disclosure, polyesters such as PET, etc. are degraded in an environmentally friendly manner, and at the same time, degradation products with high commercial value, such as mono(2-hydroxymethyl) terephthalate (MHET), terephthalic acid (TPA), and/or ethylene glycol (EG) are produced, which may be recycled in the production of polyesters such as PET, etc.

### [Brief Description of the Drawings]

FIG. 1 shows the results of investigating PET degradation activity and Tm value by deriving 10 PETase candidates; and
FIG. 2 shows the results of examining the PET degradation activity of CaPETase.

### [Detailed Description of Preferred Embodiments]

One aspect of the present disclosure provides a modified polypeptide having a PET degradation activity.

In one specific embodiment, i) the modified polypeptide is a polypeptide having at least 70% and less than 100%sequence identity to SEQ ID NO: 1; and/or
ii) the modified polypeptide is a polypeptide encoded by a polynucleotide having at least 70% and less than 100%sequence identity to a coding sequence of a mature polypeptide of SEQ ID NO: 1; and/or
iii) the modified polypeptide is a polypeptide encoded by a polynucleotide that hybridizes with (a) the coding sequence of the mature polypeptide of SEQ ID NO: 1, (b) cDNA thereof, or (c) the full-length complement of (a) or (b) under low stringency conditions, medium stringency conditions, medium-high stringency conditions, high stringency conditions, or very high stringency conditions; and/or
iv) the modified polypeptide is a functional fragment of the polypeptides of i) to iii) that has the PET degradation activity; and
the modified polypeptide comprises any one modification selected from the following modifications:
   deletion of amino acids, insertion of the amino acids, substitution with different amino acids, and/or a combination thereof at any one or more positions of positions 155 and 109;
   wherein, the position number is a position corresponding to the position of the polypeptide of SEQ ID NO: 1.

In any one specific embodiment of the specific embodiments, before modification, the amino acid at position 155 may be alanine (A); and/or the amino acid at position 109 may be asparagine (N).

In any one specific embodiment of the specific embodiments, the modified polypeptide may comprise substitution at any one or more positions selected from the following positions:
155;
109;
155 and 109;
wherein, the position number is a position corresponding to the position of the polypeptide of SEQ ID NO: 1.

In any one specific embodiment of the specific embodiments, the modified polypeptide may comprise any one or more substitutions of the following substitutions:
a substitution of the amino acid corresponding to position 155 with arginine, leucine, methionine, valine, or serine; and
a substitution of the amino acid corresponding to position 109 with alanine, isoleucine, valine, or glutamine;
wherein, the position number is a position corresponding to the position of the polypeptide of SEQ ID NO: 1.

In any one specific embodiment of the specific embodiments, the modified polypeptide may comprise any one or more substitutions selected from the following substitutions:
a substitution of the amino acid corresponding to position 155 with arginine, leucine, methionine, valine, or serine; and
a substitution of the amino acid corresponding to position 109 with alanine, isoleucine, valine, or glutamine;
wherein, the position number is a position corresponding to the position of the polypeptide of SEQ ID NO: 1.

In any one specific embodiment of the specific embodiments, the modified polypeptide may comprise any one or more modifications selected from the following modifications:
i) a modification of alanine at position 155 with arginine, leucine, methionine, valine, or serine;
ii) a modification of asparagine at position 109 with alanine, isoleucine, valine, or glutamine; and
iii) a modification of alanine at position 155 with arginine and a modification of asparagine at position 109 with alanine:
   wherein, the position number is a position corresponding to the position of the polypeptide of SEQ ID NO: 1.

Another aspect of the present disclosure provides a composition comprising the modified polypeptide having the PET degradation activity.

In any one specific embodiment of the specific embodiments, the composition may be a composition for degrading PET.

Still another aspect of the present disclosure provides a polynucleotide encoding the modified polypeptide.

Still another aspect of the present disclosure provides a nucleic acid construct comprising the polynucleotide.

Still another aspect of the present disclosure provides a vector comprising the polynucleotide or the nucleic acid construct.

Still another aspect of the present disclosure provides a host cell comprising the modified polypeptide, the polynucleotide, the nucleic acid construct, and/or the vector.

Still another aspect of the present disclosure provides a method of preparing the modified polypeptide, the method comprising the steps of culturing the host cell; and recovering the modified polypeptide expressed in the culturing step.

Still another aspect of the present disclosure provides a method of degrading a polyester, the method comprising treating the polyester with the modified polypeptide; the host cell expressing the polypeptide; and/or the composition comprising the modified polypeptide.

In any one specific embodiment of the specific embodiments, the polyester may be PET.

Still another aspect of the present disclosure provides a method of producing mono(2-hydroxymethyl) terephthalate (MHET), terephthalic acid (TPA), and/or ethylene glycol (EG), the method comprising bringing a polyester into contact with the modified polypeptide; the host cell expressing the polypeptide; and/or the composition comprising the polypeptide.

In any one specific embodiment of the specific embodiments, the polyester may be PET.

In any one specific embodiment of the specific embodiments, the step of recovering MHET, TPA, and/or EG produced by the above method may be further comprised.

Still another aspect of the present disclosure may comprise the step of synthesizing the polyester using the produced MHET, TPA, and/or EG.

In any one specific embodiment of the specific embodiments, the polyester may be PET.

Still another aspect of the present disclosure provides use of the modified polypeptide, the host cell expressing the modified polypeptide, and/or the composition comprising the modified polypeptide in degrading PET.

Still another aspect of the present disclosure provides use of the modified polypeptide or the composition comprising the modified polypeptide for the reaction with a polyester in producing mono(2-hydroxymethyl) terephthalate (MHET), terephthalic acid (TPA), and/or ethylene glycol (EG).

### [Mode for Carrying Out the Invention]

The present disclosure will be described in detail as follows. Meanwhile, each description and embodiment disclosed in the present disclosure may also be applied to other descriptions and embodiments. That is, all combinations of various elements disclosed in the present disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

As used in the specification and appended claims of the present disclosure, the singular forms ("a", "an", and "the") include plural referents unless the context clearly states otherwise. Unless the context indicates otherwise, singular terms shall comprise the plural, and plural terms shall comprise the singular. As used in the specification and appended claims of the present disclosure, unless stated otherwise, the use of "or" may be used to comprise "and/or".

As used herein, the term "about" may be presented before a particular numerical value. The term "about" used herein comprises not only the exact number recited after the term, but also a range that is near or close to that number. Considering the context in which the number is presented, it may be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

As used herein, the descriptions such as the terms "first, second, third..." "i), ii), iii)..." or "(a), (b), (c), (d)..." are used to distinguish similar elements, and these terms do not mean that the elements are performed continually or sequentially. For example, when the terms are used in reference to steps of a method, use, or assay, there may be no time interval between these steps, or they may be performed concurrently, or may be performed several seconds, several minutes, several hours, several days, or several months apart.

As used herein, the term "consisting essentially of" may mean that, when the features of the object claimed herein are not substantially affected by the presence of an unspecified component, the unspecified component may be present.

As used herein, the term "consisting of" means that the total ratio of specific component(s) is 100%. The components or features that are recited after the term "consisting of" may be essential or mandatory. In some embodiments, in addition to the components or features recited after the term "consisting of", any other components or non-essential components may be excluded.

As used herein, the term "comprising" means the presence of features, steps or components recited after the term, and does not exclude the presence or addition of one or more features, steps or components. The components or features recited after the term "comprising" herein may be essential or mandatory. However, in some embodiments, the term may further comprise any other or non-essential components or features.

As used herein, the term "comprising" may be modified to refer to "consisting essentially of" or "consisting of" in some embodiments.

With respect to amino acid sequences in the present disclosure, although it is described as a polypeptide "comprising" an amino acid sequence described by a specific sequence number, a polypeptide "consisting of" an amino acid sequence described by a specific sequence number, or a polypeptide or protein "having" an amino acid sequence described by a specific sequence number, it is apparent that any protein having an amino acid sequence in which part of the sequence is deleted, modified, substituted, conservatively substituted or added may be used in the present disclosure if it has the identical or corresponding activity to that of the polypeptide consisting of the amino acid sequence of the corresponding sequence number. For example, it may be a case where the N-terminus and/or C-terminus of the amino acid sequence is added with a sequence that does not alter the function of the protein, a naturally occurring mutation, a silent mutation thereof, or a conservative substitution, but is not limited thereto.

As used herein, the term "protein" or "polypeptide" refers to a polymer or oligomer of consecutive amino acid residues. In the present disclosure, "polypeptide," "protein," and "peptide" may be used interchangeably with "amino acid sequence."

In some cases, an amino acid sequence exhibiting activity may be referred to as an "enzyme". In the present disclosure, unless indicated otherwise, amino acid sequences are described in an N-terminal to C-terminal direction.

As used herein, the term "recombinant" with respect to a cell, or nucleic acid, polypeptide, or vector indicates that the cell, nucleic acid, polypeptide, or vector has been modified by the introduction of a heterologous nucleic acid or polypeptide or the alteration of a native nucleic acid or polypeptide, or that the cell is derived from a cell thus modified. Therefore, for example, recombinant cells may express genes that are not found within the native (non-recombinant) form of the cells, or may express native genes that are otherwise abnormally expressed, under-expressed or not expressed at all.

As used herein, the term "isolated" refers to a substance which exists in a non-naturally occurring environment or does not exist naturally. It comprises a substance (sequence, enzyme or nucleic acid) that has been at least substantially free from a substance which is naturally associated and found in nature, for example, at least one other component having a sequence, enzyme or nucleic acid.

For example, an isolated sequence, enzyme, or nucleic acid provided herein may be provided in a form that is substantially free of one or more contaminants.

Examples of isolated substances comprise i) any substance that is non-naturally occurring, ii) any substance (e.g., enzyme, variant, nucleic acid, protein, peptide, or cofactor) from which one, or more, or all naturally-occurring components associated in nature have been removed, iii) any substance that has been artificially modified from a substance found in nature, or iv) any substance that has been modified to alter the amount of that substance relative to other components associated in nature (e.g., increase in the copy number of a gene encoding a specific substance; modification of a promoter naturally linked to a gene encoding a specific substance into a highly active promoter, etc.), but are not limited thereto.

As used herein, the term "wild-type" refers to a naturally-occurring state without artificial modification. When the term "wild-type" is used in reference to a polypeptide, it refers to a naturally occurring polypeptide, which does not have artificial mutations (substitutions, insertions, deletions, etc.) in one or more amino acid positions. Similarly, when the term "wild-type" is used in reference to a polynucleotide, it means not having artificial modifications (substitutions, insertions, deletions) in one or more nucleotides. However, polynucleotides encoding wild-type polypeptides are not limited to naturally occurring polynucleotides, and comprise sequences encoding any wild-type polypeptides.

As used herein, a parent sequence or backbone refers to a reference sequence in which modification is introduced to be a modified polypeptide. That is, the parent sequence may serve as a starting sequence in which modification such as substitutions, additions, and/or deletions may be introduced. The parent sequence may be naturally-occurring or wild-type, or a variant in which one or more substitutions, insertions or deletions have occurred in the natural or wild type, or may be an artificially synthesized sequence. When the parent sequence is an amino acid sequence exhibiting activity, i.e., an amino acid sequence of an enzyme, it may be referred to as a parent enzyme.

As used herein, the term "reference sequence" means a sequence used to determine the position of amino acids within any amino acid sequence. Any amino acid sequence may be aligned with a reference sequence to determine the position of an amino acid that corresponds to a particular position in the reference sequence within any amino acid sequence.

With respect to amino acid or nucleic acid sequences in the present disclosure, the term "fragment" means a part of a parent sequence. For example, it may be a polypeptide in the form in which one or more amino acids of the parent sequence are removed from the C- or N-terminus.

As used herein, the term "fragment" of an enzyme may refer to a "functional fragment". The "functional fragment" may also be referred to as an active fragment, and means a polypeptide that is a part of a parent enzyme and has enzymatic activity of the parent enzyme. For example, the functional fragment of an enzyme may comprise a catalytic site of the enzyme.

The fragment of the enzyme may comprise a part of the full length of the parent enzyme. For example, the fragment of the enzyme may comprise amino acids of at least about 20%, about 30%, about 40%, about 50%, about 60%, about 70%, about 80%, about 90%, about 95%, about 98%, about 99% or more, or about less than 100% of the full length of the parent enzyme, but is not limited thereto.

As used herein, the term "modifying" means changing or altering. This may be an alteration from a naturally occurring state. For example, an enzyme may be altered in such a way that the enzyme is altered from a parent sequence or a reference sequence.

In the present disclosure, a modified enzyme may be an enzyme that does not exist as it is in nature, i.e., non-naturally occurring.

As used herein, the term "modified" means alteration, e.g., from its naturally occurring form. The modified enzyme of the present disclosure comprises non-naturally occurring enzymes or naturally occurring variants. For example, the modified enzyme of the present disclosure is a modified enzyme that has not been found in nature. For example, the modified enzyme of the present disclosure may be an enzyme that does not occur spontaneously, but is not limited thereto.

As used herein, the term "modification", when used with respect to amino acid/nucleic acid sequences, may comprise substitution of an amino acid/nucleic acid residue of a parent sequence for a different amino acid/nucleic acid residue in one or more positions in an amino acid sequence, deletion of an amino acid/nucleic acid residue (or series of amino acid/nucleic acid residues) of a parent sequence in one or more positions, insertion of an amino acid/nucleic acid residue (or series of amino acid/nucleic acid residues) of a parent sequence in one or more positions, or truncation of the N-terminal and/or C-terminal amino acid sequences or 5' and/or 3' nucleic acid sequences, and any combination thereof.

As used herein, the term "variant" or "modified polypeptide" of an enzyme refers to a protein having one or more amino acids different from the parent enzyme by conservative substitution and/or other modifications. The "variant" or "modified polypeptide" may be used interchangeably. The variant or modified polypeptide may be non-naturally occurring, but is not limited thereto.

The variant differs from the sequence of the parent enzyme by one or more modifications, e.g., substitutions, deletions, and/or insertions of amino acids.

Such variants may generally be identified by modifying one or more amino acids from the parent enzyme and evaluating the properties of the modified protein. In other words, the abilities of the variants may be enhanced, unchanged, or reduced, as compared to the parent enzyme.

Further, some variants may comprise modified polypeptides in which one or more regions, such as an N-terminal leader sequence or transmembrane domain, have been removed.

Other variants may comprise those in which a part has been removed from the N- and/or C-terminal of a mature protein.

The term "variant" or "modified polypeptide" may be used interchangeably with terms such as modification, modified protein, mutant, mutein, divergent, variant, etc., and is not limited as long as the terms are used to indicate mutation.

The variants may also comprise deletion or addition of amino acids that have minimal influence on the properties and secondary structure of a polypeptide. For example, the polypeptide may be conjugated with an N-terminal signal (or leader) sequence of a protein involved in the transfer of proteins co-translationally or post-translationally. Further, the polypeptides may also be conjugated with another sequence or linker to identify, purify, or synthesize the polypeptides.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue.

Throughout the entire specification of the present disclosure, the conventional one-letter and three-letter codes for naturally occurring amino acids are used. Further, the amino acids mentioned as abbreviations herein are described according to the nomenclature rules of IUPAC-IUB as follows:

| | |
|---|---|
| Alanine Ala, A | Arginine Arg, R |
| Asparagine Asn, N | Aspartic acid Asp, D |
| Cysteine Cys, C | Glutamic acid Glu, E |
| Glutamine Gln, Q | Glycine Gly, G |
| Histidine His, H | Isoleucine Ile, I |
| Leucine Leu, L | Lysine Lys, K |
| Methionine Met, M | Phenylalanine Phe, F |
| Proline Pro, P | Serine Ser, S |
| Threonine Thr, T | Tryptophan Trp, W |
| Tyrosine Tyr, Y | Valine Val, V |

Meanwhile, any amino acid may be described as Xaa or X.

Further, three-letter codes generally allowed not only for naturally occurring amino acids, but also for other amino acids, such as Aib (2-aminoisobutyric acid), Sar (N-methylglycine), α-methyl-glutamic acid, etc. may be used.

Amino acids may be generally classified based on similarity of polarity, charge, solubility, hydrophobicity, hydrophilicity, and/or amphipathic nature of a residue. Accordingly, amino acid substitution may generally occur based on similarity of polarity, charge, solubility, hydrophobicity, hydrophilicity, and/or amphipathic nature of a residue.

For example, among the amino acids having an electrically charged side chain (electrically charged amino acids), positively charged (basic) amino acids comprise arginine, lysine, and histidine, negatively charged (acidic) amino acids comprise glutamic acid and aspartic acid; among the amino acids having an uncharged side chain (uncharged amino acids), non-polar amino acids comprise glycine, alanine, valine, leucine, isoleucine, methionine, phenylalanine, tryptophan, and proline, polar or hydrophilic amino acids comprise serine, threonine, cysteine, tyrosine, asparagine, and glutamine, and among the non-polar amino acids, aromatic amino acids comprise phenylalanine, tryptophan, and tyrosine.

As used herein, the term "gene" means a polynucleotide that encodes a polypeptide and a polynucleotide comprising regions the upstream and downstream of the coding region. In some embodiments, a gene may have a sequence (intron) inserted between each coding region (exon).

As used herein, the term "homology" or "identity" refers to a degree of relevance between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and may be used with a default gap penalty established by the program being used. Substantially, homologous or identical sequences may generally hybridize under moderately or highly stringent conditions to the full length of the sequence or at least about 50%, about 60%, about 70%, about 80%, or about 90% of the full-length. It is apparent that hybridization also comprises hybridization of a polynucleotide with a polynucleotide comprising a general codon or a codon in consideration of codon degeneracy.

Whether any two polynucleotide or polypeptide sequences have homology, similarity, or identity may be, for example, determined using a known computer algorithm such as the "FASTA" program using default parameters, as in Pearson et al (1988) [Proc. Natl. Acad. Sci. USA 85]: 2444. Alternatively, it may be determined by the Needleman Wunsch algorithm (Needleman and Wunsch, 1970, J. Mol. Biol. 48: 443-453), as performed using the Needleman program of the EMBOSS package (EMBOSS: The European Molecular Biology Open Software Suite, Rice et al., 2000, Trends Genet. 16: 276-277) (version 5.0.0 or later) (GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ET al.](1988) SIAM J Applied Math 48: 1073). For example, the homology, similarity, or identity may be determined using BLAST or ClustalW of the National Center for Biotechnology Information, but is not limited thereto.

The homology, similarity, or identity of polynucleotides or polypeptides may be, for example, determined by comparing sequence information using, for example, the GAP computer program, such as Needleman et al. (1970), J Mol Biol. 48:443, as disclosed in Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. Default parameters for the GAP program may comprise (1) a unitary matrix (comprising a value of 1 for identities and 0 for non-identities) and the weighted comparison matrix of Gribskov et al(1986) Nucl. Acids Res. 14: 6745, as disclosed in Schwartz and Dayhoff, eds., Atlas Of Protein Sequence And Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide sequences have homology, similarity, or identity with each other may be identified by comparing the sequences in a Southern hybridization experiment under stringent conditions as defined, and appropriate hybridization conditions defined are within the skill of the art, and may be determined by a method well known to those skilled in the art (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York), but is not limited thereto.

As used herein, the term "mature polypeptide" means a polypeptide in a form without a signal sequence or propeptide sequence. A mature protein/polypeptide/peptide may be a functional form of a protein/polypeptide/peptide. The mature polypeptide may be in a final form after translation or post-translational modification. Examples of the posttranslational modification comprise N-terminal or C-terminal modifications, glycosylation, phosphorylation, leader sequence removal etc., but are not limited thereto.

As used herein, the term "nucleic acid construct" refers to a single- or doublestranded nucleic acid molecule, which comprises one or more regulatory sequences, and which is artificially synthesized, or engineered to comprise a specific sequence in a manner that does not exist in nature, or isolated from nature.

As used herein, the term "expression" comprises any step involved in the production of a polypeptide, e.g., transcription, post-transcriptional modification, translation, post-translational modification, and secretion, etc., but is not limited thereto.

As used herein, the term "expression vector" refers to a linear or circular nucleic acid molecule comprising a coding sequence and an operably linked regulatory sequence for expression thereof.

As used herein, the term "operably linked" refers to a configuration of placing a regulatory sequence to an appropriate position to direct expression of a coding sequence. Thus, the term "operably linked" comprises an attachment or linking between a regulatory region of a functional domain having a known or desired activity such as a promoter, a terminator, a signal sequence, or an enhancer, and a target (gene or polypeptide) such that the expression, secretion, or function of the target may be regulated in accordance with the known or desired activity.

As used herein, the term "cDNA" refers to a DNA sequence which may be prepared by reverse transcription from a mature, spliced mRNA molecule obtained from a eukaryotic or prokaryotic cell. The cDNA sequence lacks intron sequences that may be present in the corresponding genomic DNA. The initial primary RNA transcript is a precursor to mRNA which is processed through a series of steps comprising splicing before appearing as mature spliced mRNA.

As used herein, the term "regulatory sequence" refers to a polynucleotide sequence necessary for the expression of a coding sequence. Each regulatory sequence may be native (derived from the same origin) or foreign (derived from different genes) to the coding sequence. Examples of the regulatory sequence may comprise a leader sequence, a polyadenylation sequence, a pro-peptide sequence, a promoter, a signal peptide sequence, an operator sequence, a sequence encoding a ribosome-binding domain, and a sequence regulating the termination of transcription and translation. The minimum units of the regulatory sequence may comprise a promoter, and a sequence for terminating transcription and translation.

To describe the variant provided in the present disclosure, the following nomenclature is used.

In the present disclosure, referring to a specific position in an amino acid sequence may comprise referring to an amino acid present or substituted at that position. Referring to an amino acid at a specific position may be described in various ways. For example, the "position 003" may be described as "position 3", "amino acid 3", "3rd amino acid". Further, for example, when the amino acid at position 3 is serine (S), it may be described as "S3" or "Ser3".

Amino acid substitution may be expressed by describing the amino acid before substitution, the position, and the amino acid to be substituted in sequence. The amino acids may be expressed using the conventional one-letter and three-letter codes. For example, when alanine, an amino acid at position 8 of a specific sequence, is substituted with valine, it may be described as "A8V" or "Ala8Val".

Any amino acid at a specific position may be referred to as "X". For example, X6 refers to any amino acid at position 6. In addition, when the amino acid to be substituted is expressed as X, it means that it is substituted with an amino acid different from the amino acid present before substitution. For example, "V6X" indicates that V at position 6 is substituted with any amino acid other than V.

Different alterations may be expressed by simultaneously describing several types of amino acids using symbols, such as ",". For example, when an amino acid (D) at position 12 is substituted with S or K, it may be described as D12S,K.

Multiple mutations may be described using "+" or "/". For example, descriptions such as "G2A+M8V" mean that glycine, an amino acid at position 2, is substituted with alanine, and methionine, an amino acid at position 8, is substituted with valine, respectively. For example, L180C/A202C/R242C/S291C means that leucine, an amino acid at position 180, is substituted with cysteine, alanine, an amino acid at position 202, is substituted with cysteine, arginine, an amino acid at position 242, is substituted with cysteine, and serine, an amino acid at position 291, is substituted with cysteine.

As used herein, the term "corresponding to" refers to an amino acid residue at the position recited in a protein or polypeptide, or an amino acid residue which is similar, identical, or homologous to the residue recited in a protein or polypeptide. Identifying an amino acid at a corresponding position may be determining a particular amino acid in a sequence that refers to a particular sequence. As used herein, the "corresponding region" generally refers to a similar or corresponding position in the related protein or reference protein.

In the present disclosure, SEQ ID NO: 1 may be used as a reference sequence to determine the position of amino acids in any amino acid sequence.

In other words, SEQ ID NO: 1 disclosed herein may be used to determine the corresponding amino acid residues in any polypeptide having PET degradation activity. Unless indicated otherwise in the present disclosure, residues of a particular amino acid sequence are numbered, based on SEQ ID NO: 1.

For example, any amino acid sequence is aligned with SEQ ID NO: 1, and based on the alignment, each amino acid residue of the amino acid sequence may be numbered with reference to the numerical position of the amino acid residue corresponding to the amino acid residue of SEQ ID NO: 1. For example, a sequence alignment algorithm such as that described herein may identify the position of an amino acid or a position where modifications such as substitutions, insertions, or deletions occur, compared to a query sequence (also referred to as a "reference sequence").

In such alignment, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000), Trends Genet. 16: 276-277), etc. may be used, but is not limited thereto.

Further, the corresponding amino acid residue in another PETase may be identified by multiple sequence alignment. Examples of the multiple sequence alignment known in the art comprise programs such as MUSCLE (multiple sequence comparison by log-expectation; version 3.5 or later; Edgar, 2004, Nucleic Acids Research 32: 1792-1797), MAFFT (version 6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), and EMBOSS EMMA employing ClustalW (1.83 or later; Thompson et al., 1994, Nucleic Acids Research 22 : 4673-4680), and their respective default parameters may be used, but are not limited thereto.

In addition, when enzymes diverged from the mature polypeptide of SEQ ID NO: 1 fail to detect their relationship by traditional sequence-based comparison, other pairwise sequence comparison algorithms may be used (Lindahl and Elofsson, 2000, J. Mol. Biol. 295: 613-615). Greater sensitivity in sequence-based searching may be attained using search programs that utilize probabilistic representations of polypeptide families (profiles) to search databases. For example, the PSI BLAST program generates profiles through an iterative database search process and is capable of detecting remote homologs (Atschul et al., 1997, Nucleic Acids Res. 25: 3389-3402). Even greater sensitivity may be achieved if the family or superfamily for the polypeptide has one or more representatives in the protein structure databases. Programs such as GenTHREADER (Jones, 1999, J. Mol. Biol. 287: 797-815; McGuffin and Jones, 2003, Bioinformatics 19: 874-881) utilize information from a variety of sources, such as PSI BLAST, secondary structure prediction, structural alignment profiles, and solvation potentials, as input to a neural network that predicts the structural fold for a query sequence. Similarly, the method of Gough et al., 2000, J. Mol. Biol. 313: 903-919 may be used to align a sequence of unknown structure with the superfamily models present in the SCOP database. These alignments may be used in sequence to generate homology models for the polypeptides, and such models may be assessed for accuracy using a variety of tools developed for that purpose.

For proteins of known structure, several tools and resources are available for retrieving and generating structural alignments. For example, the SCOP superfamilies of proteins have been structurally aligned, and those alignments are accessible and downloadable. Two or more protein structures may be aligned using a variety of algorithms such as the distance alignment matrix (Holm and Sander, 1998, Proteins 33: 88-96) or CE (Combinatorial extension) Shindyalov and Bourne, 1998, Protein Engineering 11: 739-747). Implementation of these algorithms may additionally be utilized to query structure databases with a structure of interest in order to discover possible structural homologs (Holm and Park, 2000, Bioinformatics 16: 566-567).

The above methods are one exemplary, and are not limited thereto.

Hereinafter, the specific embodiments of the present disclosure will be described in more detail below.

In the present disclosure, the "polypeptide having PET degradation activity" or "PETase" is a polypeptide having depolymerization activity for polyethylene terephthalate (PET) and may also comprise a polypeptide having depolymerization activity for an oligomer obtained by depolymerization of PET, e.g., for bis(2-hydroxyethyl) terephthalate (BHET). The term "depolymerization" means a process whereby a polymer or at least one polymer of the plastic material is depolymerized into smaller molecules, such as monomers and/or oligomers.

In the present disclosure, the PET degradation activity may be measured and evaluated using methods known in the art by comprising the embodiments described herein, for example, may be evaluated by measuring the production amount of BHET, MHET, TPA, or EG.

As used herein, the term "parent PETase" refers to PETase to which a modification is applied in order to produce the variant or modified polypeptide of the present disclosure. Specifically, the parent PETase, parent enzyme, or parent sequence may be a naturally occurring polypeptide or a wild-type polypeptide, may be a mature polypeptide thereof, and may comprise a variant or functional fragment thereof, but is not limited thereto, as long as the polypeptide has PETase activity and may become a parent of the variant.

The parent PETase provided in the present disclosure may be a polypeptide of SEQ ID NO: 1, but is not limited thereto. Further, it may be a polypeptide having about 60%, 70%, 75%, 80%, 85%, 90%, 95%, 96%, 97%, 98% or 99% or more sequence identity to the polypeptide of SEQ ID NO: 1 as long as it has the PET degradation activity, and any polypeptide may be comprised within the scope of the parent PETase as long as it has the identical or corresponding activity to that of the polypeptide consisting of the amino acid sequence of SEQ ID NO: 1.

The parent PETase of the variant provided herein may be derived from a microorganism of *Cryptosporangium* sp., specifically, *Cryptosporangium aurantiacum.*

Meanwhile, the above-described microorganism is an exemplary microorganism from which the parent PETase provided herein may be derived, and comprises those derived from microorganisms taxonomically homologous to the microorganism regardless of the name of the microorganisms.

The above-described microorganism may be obtained from known microorganism depositary authorities such as ATCC, DSMZ, CBS, NRRL, KCTC, KCCM.

As used herein, a sequence "derived from" a specific microorganism is not limited to those naturally produced or producible in that microorganism, but also comprises sequences encoded by genes which are produced and isolated from the microorganism comprising the genes.

For example, PETase derived from *Cryptosporangium* sp. may comprise not only enzymes having PETase activity naturally produced in *Cryptosporangium* sp., but also those produced in *Cryptosporangium* sp. source, and those produced in other host cells through genetic modifications known in the art (e.g., transformation into a sequence encoding the enzyme).

Further, in the present disclosure, it was newly investigated that the polypeptide of SEQ ID NO: 1 has the PET degradation activity, and a modified polypeptide having altered or enhanced characteristics was prepared by introducing mutations into the parent sequence of SEQ ID NO: 1. The description of the parent PETase of the variant provided in the present disclosure may be applied to the polypeptide having the identical or corresponding activity to the polypeptide consisting of the amino acid sequence of SEQ ID NO: 1.

As used herein, the "modified polypeptide having PET degradation activity" may be a variant of the parent PETase.

As used herein, the term "variant of PETase" or "PETase variant" refers to a protein having PET degradation activity with one or more amino acids different from the amino acid sequence of the parent PETase.

The "modified polypeptide having PET degradation activity", "variant of PETase" and "PETase variant" may be used interchangeably.

The variant provided in the present disclosure may comprise one or more amino acid modifications in the sequences of the parent PETase, while having PETase activity. The modification may be deletion of amino acids, insertion of amino acids, substitution with another amino acid, and/or a combination thereof, specifically, substitution of amino acids.

Further, the variant may be i) a polypeptide having at least 70% and less than 100% sequence identity to SEQ ID NO: 1; and/or ii) the variant may be a polypeptide encoded by a polynucleotide having at least 70% and less than 100% sequence identity to the coding sequence of the mature polypeptide of SEQ ID NO: 1; and/or iii) the variant may be a polypeptide encoded by a polynucleotide that hybridizes with (a) the coding sequence of the mature polypeptide of SEQ ID NO: 1, (b) cDNA thereof, or (c) the full-length complement of (a) or (b) under low stringency conditions, medium stringency conditions, medium-high stringency conditions, high stringency conditions, or very high stringency conditions; and/or iv) the variant may be a functional fragment of the polypeptide of i), ii), or iii) that has the PET degradation activity.

Specifically, the variant provided in the present disclosure comprises modification of one or more amino acids in the parent PETase sequence while having PET degradation activity, thereby having one or more altered functions or properties, as compared to the parent PETase.

In one specific embodiment, the variant provided in the present disclosure comprises modification of one or more amino acids in the parent PETase sequence while having PET degradation activity, thereby having one or more altered functions or properties, as compared to the parent PETase, and having one or more conservative substitutions.

The variant provided in the present disclosure, which is a variant of the parent PETase, may be a polypeptide having PET degradation activity.

In one specific embodiment, the variant provided in the present disclosure may comprise modifications at one or more positions corresponding to positions 155 and 109 of SEQ ID NO: 1. Specifically, the modifications may be deletion of amino acids, insertion of amino acids, substitution with another amino acid, and/or a combination thereof, specifically, substitution of amino acids.

In the present disclosure, the position number is a position corresponding to the position of the polypeptide of SEQ ID NO: 1, and the term "corresponding" is as described above.

In any one specific embodiment of the aforementioned specific embodiments, the variant provided in the present disclosure may comprise amino acid modifications corresponding to one or more of A155 and N109 of SEQ ID NO: 1.

In any one specific embodiment of the aforementioned specific embodiments, with regard to SEQ ID NO: 1 before modification, which is provided in the present disclosure, the amino acid at position 155 may be alanine (A); and/or the amino acid at position 109 may be asparagine (N).

In any one specific embodiment of the aforementioned specific embodiments, the variant provided in the present disclosure may comprise a substitution of the amino acid corresponding to position 155 of SEQ ID NO: 1 with G, V, L, I, M, F, W, P, S, T, C, Y, N, Q, D, E, K, R, or H, and specifically, with R, L, M, V, or S.

In any one specific embodiment of the aforementioned specific embodiments, the variant provided in the present disclosure may comprise a substitution of the amino acid corresponding to position 109 of SEQ ID NO: 1 with G, A, V, L, I, M, F, W, P, S, T, C, Y, Q, D, E, K, R, or H, and specifically, with A, I, V or Q.

In any one specific embodiment of the aforementioned specific embodiments, the variant provided in the present disclosure may comprise any one or more substitutions of the following substitutions:
A155R; A155L; A155M; A155V; A155S; N109A; N109I; N109V; and N109Q.

In any one specific embodiment of the aforementioned specific embodiments, the variant provided in the present disclosure may comprise any one or more substitutions of the following substitutions:
a substitution of the amino acid corresponding to position 155 with arginine, leucine, methionine, valine, or serine; and
a substitution of the amino acid corresponding to position 109 with alanine, isoleucine, valine, or glutamine.

In any one specific embodiment of the aforementioned specific embodiments, the variant provided in the present disclosure may comprise a substitutions of the amino acid corresponding to position 155 with arginine and a substitution of the amino acid corresponding to position 109 with alanine in the amino acid sequence of SEQ ID NO: 1.

In any one specific embodiment of the aforementioned specific embodiments, the variant provided in the present disclosure comprises all possible combinations of the above-described modifications.

In any one specific embodiment of the aforementioned specific embodiments, the variant provided in the present disclosure may have about 60% or more, for example, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more, and less than 100% sequence identity to the parent PETase; a mature polypeptide thereof, or a functional fragment thereof.

In any one specific embodiment of the aforementioned specific embodiments, the variant provided in the present disclosure may have about 60% or more, for example, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more, and less than 100% sequence identity to SEQ ID NO: 1.

In any one specific embodiment of the aforementioned specific embodiments, the variant provided in the present disclosure may be a polypeptide encoded by a polynucleotide having about 60% or more, for example, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more, and less than 100% sequence identity to the coding sequence of the mature polypeptide of SEQ ID NO: 1.

In any one specific embodiment of the aforementioned specific embodiments, the variant provided in the present disclosure may have about 60% or more, for example, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more, and less than 100% sequence identity to the functional fragment of SEQ ID NO: 1.

In the variant polypeptide provided in the present disclosure, one or more of any selectable or detectable properties or attributes of a polypeptide may be altered as compared to other parent PETases, e.g., a wild-type PETase, parent PETase, other PETase variants, etc.

The properties or attributes may comprise, but are not limited to, oxidative stability, substrate specificity, catalytic activity, thermal stability, alkaline stability, pH activity profile, resistance to proteolytic degradation, Km, k_{cat}, k_{cat}/Km ratio, protein folding, inducing an immune response, ability to bind to a ligand, ability to bind to a receptor, ability to be secreted, ability to be displayed on the surface of a cell, ability to oligomerize, ability to signal, ability to stimulate cell proliferation, ability to inhibit cell proliferation, ability to induce apoptosis, ability to be modified by phosphorylation or glycosylation, and/or ability to treat diseases, etc.

Specifically, the variant provided in the present disclosure may have any one or more of the following modified activities, as compared to the parent sequence:
i) increased or decreased enzymatic activity;
ii) increased or decreased specific activity;
iii) increased or decreased pH stability;
iv) increased or decreased storage stability;
v) increased or decreased acid tolerance;
vi) increased or decreased thermal tolerance; and
vii) altered substrate specificity; but is not limited thereto.

For another example, the PETase provided in the present disclosure may have any one or more of the following modified activities, as compared to PETase derived from *Ideonella sakaiensis* or PETase derived from *Thermobifida fusca:*
i) increased or decreased enzymatic activity;
ii) increased or decreased specific activity;
iii) increased or decreased pH stability;
iv) increased or decreased storage stability;
v) increased or decreased acid tolerance;
vi) increased or decreased thermal tolerance; and
vii) altered substrate specificity; but is not limited thereto.

For still another example, the PETase provided in the present disclosure may have any one or more of the following modified activities, as compared to PETase derived from *Ideonella sakaiensis* or PETase derived from *Thermobifida fusca:*
i) increased enzymatic activity;
ii) increased specific activity;
iii) increased pH stability;
iv) increased storage stability;
v) increased acid tolerance;
vi) increased thermal tolerance; and
vii) altered substrate specificity; but is not limited thereto.

As used herein, the "enzymatic activity" represents at least one catalytic activity. Specifically, it may be the conversion efficiency of an enzyme mainly expressed as kcat/Km, but is not limited thereto.

When the enzyme is completely saturated with substrates, k_{cat} means the rate constant (catalytic constant) that converts substrates into products in unit time by one enzyme, and is also referred to as a turnover number. Km is the substrate concentration when the reaction rate is at half the maximum value (Vmax).

Examples of the ways to express enzymatic activity comprise specific activity (umol of converted substrate x mg⁻¹ x min⁻¹) or volumetric activity (umol of converted substrate x mL⁻¹ x min⁻¹), etc.

However, defining the enzymatic activity is not limited to the description described above, and the enzymatic activity may be defined and evaluated based on the information disclosed in Irwin H. Segel, Enzyme kinetics, John Wiley & Sons, 1979; A. G. Marangoni, Enzyme kinetics, Wiley-Interscience, 2003; A. Fersht, Enzyme structure and mechanisms, John Wiley & Sons, 1981; Structure and Mechanism in Protein Science: A guide to enzyme catalysis and protein folding, Alan Fersht, W.H. Freeman, 1999; Fundamentals of Enzyme Kinetics, Athel Cornish-Bowden, Wiley-Blackwell 2012 and Voet et al., "Biochemie" [Biochemistry], 1992, VCH-Verlag, Chapter 13, pages 331-332 with respect to enzymatic activity, etc.

In one specific embodiment, the variant provided in the present disclosure may have an increased enzymatic activity by about 100%, about 110%, about 120%, about 130%, about 140%, about 150%, about 160%, about 170%, about 180%, about 190%, or about 200% or more, as compared to the parent enzyme.

In another specific embodiment, the variant provided in the present disclosure may have a decreased enzymatic activity by about 99%, about 95%, about 90%, about 80%, about 70%, about 60%, about 50%, about 40%, or about 20% or less, as compared to the parent enzyme.

As used herein, the term "specific activity" is the activity of an enzyme per unit weight of proteins, and may be expressed as unit/mg. The quantification of proteins may be performed using, for example, SDS-PAGE or Bradford assay.

The enzymatic stability means that enzyme activity is maintained during storage or reaction time. In order to measure such changes in stability, the initial enzyme activity may be measured and compared under defined conditions at time zero (100%) and after a predetermined period of time (x%), and thus, the level, at which the enzyme activity is lost, or the enzyme stability may be expressed.

Factors that affect enzymatic activity comprise, for example, pH, heat, the presence of other substances (e.g., oxidizing agents, chelating agents), etc.

As used herein, the term "pH stability" means the ability of a protein to function in a specific pH range. In one specific embodiment, the variant provided in the present disclosure may have activity at about pH 4.0 to about pH 12.0, but is not limited thereto.

When a protein maintains its function in a specific pH range, it may be defined as having "pH stability", and may also be defined as having "acid tolerance", "alkali tolerance", etc., according to the pH range.

As used herein, the term "thermal stability" means the ability of a protein to function in a specific temperature range. In one specific embodiment, the variant provided in the present disclosure may have activity in the range of about 20°C to about 70°C, specifically, in the range of about 25°C to about 65°C, but is not limited thereto.

As used herein, the term "thermal tolerance" means the ability of a protein to function after exposure to a specific temperature, e.g., high heat or cryogenic temperature. For example, proteins having thermal tolerance may not function at a temperature they are exposed to, but may become functional when returned to an optimal temperature environment.

An increase in stability may comprise maintaining high enzymatic activity; increasing the range of pH, temperature and/or time, etc., at which a protein maintains its function, by comparing to other enzymes, e.g., a wild-type enzyme, a parent enzyme, and/or other variants.

A decrease in stability may comprise maintaining low enzymatic activity; decreasing the range of pH, temperature and/or time, etc., at which a protein maintains its function, by comparing to other enzymes, e.g., a wild-type enzyme, a parent enzyme, and/or other variants.

As used herein, the term "substrate specificity" means the ability of an enzyme to identify a substrate and molecules that compete with the substrate. Substrate specificity may be determined by measuring the activity of an enzyme for different substrates. In one specific embodiment, the change in substrate specificity may be a change in the direction of increasing specificity for a substrate capable of producing a desired product. In another specific embodiment, the change in substrate specificity may be a change in a direction of decreasing specificity for a substrate capable of producing a desired product.

The "polynucleotide" encoding the variant of the present disclosure may comprise the coding sequence of the above-described variant. The polynucleotide may undergo various modifications in the coding region within the scope that does not change the amino acid sequence of the polypeptide, due to codon degeneracy or in consideration of the codons preferred in an organism in which the polypeptide is to be expressed.

Further, the polynucleotide of the present disclosure may comprise a probe that may be prepared from a known gene sequence, for example, any sequence encoding the variant of the present disclosure by hybridizing with a sequence complementary to all or part of the nucleotide sequence under stringent conditions without limitation.

The "stringent conditions" refers to conditions under which specific hybridization between polynucleotides is allowed. Such conditions are specifically described in the literature (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York).

For example, the stringent conditions may comprise conditions under which polynucleotides having a high homology or identity of 40% or more, specifically 90% or more, more specifically 95% or more, 96% or more, 97% or more, 98% or more, and even more specifically 99% or more are hybridized with each other and polynucleotides having a homology or identity lower than the above homologies or identities are not hybridized with each other, or washing conditions of the common Southern hybridization, washing once, or specifically, twice or three times at a salt concentration and a temperature corresponding to 60°C, 1×SSC, 0.1% SDS, specifically, 60°C, 0.1×SSC, 0.1% SDS, and more specifically, 68°C, 0.1×SSC, 0.1% SDS.

Hybridization requires that two nucleic acids have complementary sequences, although mismatches between bases are possible depending on the stringency of the hybridization. The term "complementary" is used to describe the relationship between nucleotide bases that may hybridize with each other. For example, with respect to DNA, adenosine is complementary to thymine, and cytosine is complementary to guanine. Therefore, the polynucleotide of the present disclosure may comprise isolated nucleotide fragments complementary to the entire sequence as well as nucleic acid sequences substantially similar thereto.

Specifically, polynucleotides having a homology or identity may be detected using the hybridization conditions comprising a hybridization step at a Tm value of 55°C under the above-described conditions. Further, the Tm value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (see, Sambrook et al., supra, 9.50-9.51, 11.7-11.8).

For example, the "high stringency" may occur at about 5°C to 10°C below the Tm of the probe; the "medium stringency" may occur at about 10°C to 20°C below the Tm of the probe; and the "low stringency" may occur at about 20°C to 25°C below the Tm, but the stringency is not limited thereto.

For example, the "low stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 25% formamide, according to the standard Southern blotting procedures for 12 hours to 24 hours. The carrier material may be finally washed twice or three times each for 15 minutes in 2 X SSC, 0.1% to 0.2% SDS at 50°C.

For example, the "medium stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 35% formamide, according to the standard Southern blotting procedures for 12 hours to 24 hours. The carrier material may be finally washed twice or three times each for 15 minutes in 2 X SSC, 0.1% to 0.2% SDS at 55°C. For example, the "medium-high stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 35% formamide, according to the standard Southern blotting procedures for 12 hours to 24 hours. The carrier material may be finally washed twice or three times each for 15 minutes in 1 X to 2 X SSC, 0.1% to 0.2% SDS at 60°C.

For example, the "high stringency condition" may mean, for probes of at least about 100 nucleotides in length, prehybridization and hybridization at 42°C in 5×SSPE, 0.3% SDS, 200 micrograms/ml of sheared and denatured salmon sperm DNA, and 35% formamide, according to the standard Southern blotting procedures for 12 hours to 24 hours. The carrier material may be finally washed twice or three times each for 15 minutes in 2 X SSC, 0.1% to 0.2% SDS at 65°C.

The "nucleic acid construct" provided in the present disclosure comprises a polynucleotide encoding the variant provided in the present disclosure, which is operably linked to one or more regulatory sequences that direct expression of the coding sequence in a suitable host cell under conditions suitable for the regulatory sequences.

Polynucleotides may be manipulated in a variety of ways to allow expression of the variant. Depending on the expression vector, it may be desirable or necessary to manipulate the polynucleotides prior to insertion into the vector. Such manipulations may be performed using methods known in the art.

The "vector" provided in the present disclosure refers to a DNA construct comprising the nucleotide sequence of the polynucleotide encoding the variant operably linked to a suitable expression regulatory region (expression regulatory sequence) so as to be able to express the variant of the present disclosure in a suitable host cell. The expression regulatory region may comprise a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into the genome thereof.

The vector that may be used in the present disclosure is not particularly limited, and any vector known in the art may be used. Examples of the vectors commonly used may comprise natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, etc. may be used, and as a plasmid vector, those based on pBR, pUC, pBluescriptll, pGEM, pTZ, pCL and pET, etc. may be used. Specifically, pDZ, pACYC177, pACYC184, pCL, pECCG117, pUC19, pBR322, pMW118, pCC1BAC vector, etc. may be used.

For example, the polynucleotide encoding the variant provided in the present disclosure may be inserted into the chromosome through a vector for intracellular chromosomal insertion. The insertion of the polynucleotide into the chromosome may be performed by any method known in the art, for example, by homologous recombination, but is not limited thereto. The vector may further comprise a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cytotoxic agents, or expression of surface polypeptides, may be used. Only cells expressing the selection marker are able to survive or show different phenotypes under the environment treated with the selective agent, and thus the transformed cells may be selected.

The "host cell" of the present disclosure may comprise any host cell without limitation as long as it is able to express the variant of the present disclosure.

The host cell of the present disclosure may comprise the above-described variant, the polynucleotide encoding the variant, the nucleic acid construct and/or vector comprising the same.

The nucleic acid construct or vector may be integrated into the chromosome as described earlier, or may remain as a self-replicating extrachromosomal vector.

The host cell of the present disclosure includes any progeny of a parent cell that is not identical to the parent cell due to mutations that occur during replication.

The host cell may be any cell useful in the recombinant production of the variant, e.g., a prokaryotic cell or a eukaryotic cell.

The prokaryotic host cell may be any Gram-positive or Gram-negative bacterium.

The Gram-positive bacteria comprise, but are not limited to, *Bacillus, Clostridium, Enterococcus, Geobacillus, Lactobacillus, Lactococcus, Oceanobacillus, Staphylococcus, Streptococcus, Corynebacterium,* and *Streptomyces.*

The Gram-negative bacteria comprise, but are not limited to, *Campylobacter, Escherichia, Flavobacterium, Fusobacterium, Helicobacter, Ilyobacter, Neisseria, Pseudomonas, Salmonella, Vibrio* (e.g., *Vibrio natriegens),* and *Ureaplasma.*

In one specific embodiment, the bacterial host cell may be a host cell belonging to the genus *Bacillus,* specifically, comprising *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells, but is not limited thereto.

In one specific embodiment, the bacterial host cell may be a host cell belonging to the genus *Streptococcus,* specifically, comprising *Streptococcus equisimilis, Streptococcus pyogenes, Streptococcus uberis,* and *Streptococcus equi subsp. Zooepidemicus* cells, but is not limited thereto.

In one specific embodiment, the bacterial host cell may be a host cell belonging to the genus *Streptomyces,* specifically, comprising *Streptomyces achromogenes, Streptomyces avermitilis, Streptomyces coelicolor, Streptomyces griseus,* and *Streptomyces lividans* cells, but is not limited thereto.

In one specific embodiment, the bacterial host cell may be a host cell belonging to the genus *Corynebacterium,* specifically, *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* but is not limited thereto.

In one specific embodiment, the bacterial host cell may be a host cell of the genus *Escherichia,* and may be *Escherichia coli (E.coli),* but is not limited thereto.

The host cell may be a eukaryotic cell, such as a mammalian, insect, plant, or fungal cell.

The host cell may be a fungal cell. As used herein, the "fungi" may comprise *Ascomycota, Basidiomycota, Chytridiomycota,* and *Zygomycota* as well as the *Oomycota* and all Fungi *imperfecti.*

The fungal host cell may be a yeast cell. As used herein, the "yeast" comprises *ascosporogenous* yeast *(Endomycetales), basidiosporogenous* yeast, and yeast belonging to the Fungi *imperfecti (Blastomycetes).* However, this classification may change, and may be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Komagataella, Saccharomyces, Schizosaccharomyces,* or *Yarrowia* cell, for example, *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis, Komagataella phaffii,* or *Yarrowia lipolytica* cell.

The fungal host cell may be a filamentous fungal cell. The "filamentous fungi" comprise all filamentous forms of the subdivision *Eumycota* and *Oomycota* (as defined by the above literature (Hawksworth et al., 1995)). The filamentous fungi are generally characterized by a mycelial wall composed of chitin, cellulose, glucan, chitosan, mannan, and other complex polysaccharides. Vegetative growth is by hyphal elongation, and carbon catabolism is obligately aerobic. In contrast, vegetative growth by yeasts, for example, *Saccharomyces cerevisiae* is by budding of a unicellular thallus, and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell, but is not limited thereto.

The "composition" of the present disclosure may comprise the modified polypeptide having the PET degradation activity, or a host cell expressing the modified polypeptide.

The descriptions of the modified polypeptide provided in the present disclosure and the host cell expressing the same may be applied to the modified polypeptide having the PET degradation activity, or the host cell expressing the modified polypeptide.

The composition of the present disclosure may be used in converting polyester into a final product.

The term "polyester" refers to a polymer comprising an ester functional group in the main chain of its structure. For example, polyethylene terephthalate is a semi-aromatic copolymer composed of two monomers, terephthalic acid and ethylene glycol (EG).

The polyester may be selected from polyethylene terephthalate (PET), polyethylene terephthalate glycol (PETG), polyethylene co-isosorbide-terephthalate (PEIT), polytrimethylene terephthalate (PTT), polybutylene adipate terephthalate (PBAT), polycyclohexylenedimethylene terephthalate (PCT), and polybutylene terephthalate (PBT). Specifically, the polyester may be PET.

The polypeptide of the present disclosure or the composition comprising the same may be used to depolymerize PET into bis(2-hydroxyethyl) terephthalate (BHET), or to decompose PET into mono(2-hydroxymethyl) terephthalate (MHET) and terephthalic acid (TPA).

The polypeptide of the present disclosure or the composition comprising the same may be used to degrade polymers (e.g., oligomers) resulting from PET depolymerization. For example, it may be used to degrade BHET into MHET and TPA.

The composition of the present disclosure may further comprise other components, in addition to the variant having the PET degradation activity, which is provided in the present disclosure. The components added to the composition of the present disclosure may be appropriately selected by those skilled in the art.

In one specific embodiment, the composition of the present disclosure may further comprise any components suitable for being applied to conversion of PET into the final products.

In one specific embodiment, the composition of the present disclosure may further comprise any components suitable for being applied to PET degradation.

Examples of materials that may be added comprise stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, solubilizers, isotonic agents, stabilizers, diluents, lubricants, preservatives, etc., but are not limited thereto.

In one specific embodiment, the composition provided in the present disclosure may further comprise a naturally occurring material or a non-naturally occurring material, in addition to the variant provided in the present disclosure.

In one specific embodiment, the composition provided in the present disclosure may further comprise additional enzymes, in addition to the variant provided in the present disclosure.

A method of preparing the variant of the present disclosure may comprise the steps of culturing the host cell; and recovering the variant expressed in the culturing step.

As used herein, the term "culturing" means that the host cell is grown under appropriately controlled environmental conditions. The culturing process of the present disclosure may be performed in a suitable medium and culture conditions known in the art. Such a culturing process may be easily adjusted for use by those skilled in the art according to the strain to be selected. Specifically, the culture may be a batch culture, a continuous culture, and a fed-batch culture, but is not limited thereto.

As used herein, the term "medium" refers to a mixture of materials which comprise, as a main ingredient, nutrient materials required for culturing the host cell, and it supplies nutrient materials and growth factors, along with water that is essential for survival and growth. Specifically, the medium and other culture conditions used for culturing the host cell of the present disclosure may be any medium commonly used for culturing host cells without any particular limitation. However, the host cell of the present disclosure may be cultured under aerobic conditions in a common medium comprising appropriate carbon sources, nitrogen sources, phosphorus sources, inorganic compounds, amino acids, and/or vitamins, etc., while adjusting temperature, pH, etc.

In the present disclosure, the carbon sources may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; amino acids, such as glutamic acid, methionine, lysine, etc. Further, the carbon sources may comprise natural organic nutrients such as starch hydrolysate, molasses, blackstrap molasses, rice bran, cassava, sugar cane molasses, and corn steep liquor, etc. Specifically, carbohydrates such as glucose and sterilized pretreated molasses (i.e., molasses converted to reducing sugar) may be used, and in addition, various other carbon sources may be used in an appropriate amount without limitation. These carbon sources may be used alone or in combination of two or more thereof, but are not limited thereto.

The nitrogen sources may comprise inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extract, yeast extract, malt extract, corn steep liquor, casein hydrolysate, fish or decomposition product thereof, defatted soybean cake or decomposition product thereof, etc. These nitrogen sources may be used alone or in combination of two or more thereof, but are not limited thereto.

The phosphorus sources may comprise monopotassium phosphate, dipotassium phosphate, or corresponding sodium-comprising salts, etc. The inorganic compounds may comprise sodium chloride, calcium chloride, iron chloride, magnesium sulfate, iron sulfate, manganese sulfate, calcium carbonate, etc. In addition, amino acids, vitamins, and/or appropriate precursors, etc. may be comprised. These components or precursors may be added to a medium in a batch or continuous manner, but these phosphorus sources are not limited thereto.

Further, pH of the medium may be adjusted by adding, to the medium, a compound such as ammonium hydroxide, potassium hydroxide, ammonia, phosphoric acid, sulfuric acid, etc. in an appropriate manner during the culturing of the host cell. In addition, bubble formation may be prevented during the culturing using an antifoaming agent such as fatty acid polyglycol ester. Further, oxygen or a gas comprising oxygen may be injected to the medium to maintain aerobic conditions of the medium, or no gas may be injected or nitrogen, hydrogen, or carbon dioxide gas may be injected to maintain anaerobic or microaerobic conditions, but are not limited thereto.

The temperature of the medium may be 20°C to 50°C, specifically, 25°C to 40°C, but is not limited thereto. The culturing may be continued until the desired amount of a useful material is obtained, and may be specifically carried out for 24 hours to 196 hours, but is not limited thereto.

In one specific embodiment, the variant expressed in the culturing step may be recovered using methods known in the art to which the present disclosure pertains. For example, the variant may be recovered from a nutrient medium by common procedures comprising, but are not limited to, collection, centrifugation, filtration, extraction, spray-drying, evaporation, or precipitation.

The recovering method is to collect the variant using the method of culturing the host cell of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, etc., HPLC, and a combination thereof may be used, and the variant may be recovered from the medium or the host cell using suitable methods known in the art.

In another specific embodiment, the variant expressed by the host cell in the culturing step may not be recovered. In the specific embodiment, the host cell expressing the variant may be used as it is as a source of the variant.

The present disclosure may comprise a method of producing mono(2-hydroxymethyl) terephthalate (MHET), terephthalic acid (TPA), and/or ethylene glycol (EG), the method comprising bringing a polyester into contact with the above-described modified polypeptide, or the host cell expressing the polypeptide, or the composition comprising the same.

Specifically, the polyester may comprise the aforementioned descriptions.

In the present disclosure, the polyester may be degraded by bringing the polyester into "contact" with the modified polypeptide or the host cell expressing the polypeptide or the composition comprising the same, but is not limited thereto. The degradation may also be referred to as depolymerization.

The time required for degradation of the polyester may vary depending on the polyester-comprising article itself (i.e., nature and origin of the plastic product, its composition, shape etc.), the type and amount of the modified polypeptide used, as well as various process parameters (i.e., temperature, pH, additional agents, etc.). The process parameters may be easily applied to the polyester-comprising article using a technology known in the art.

For example, the degradation process may be carried out at 20°C to 90°C, preferably at 40°C to 80°C, and more preferably at 50°C to 70°C. More specifically, the temperature may be maintained below an inactivation temperature, which corresponds to the temperature at which the modified polypeptide becomes inactive and/or below a temperature at which the host cell no longer synthesizes the modified polypeptide.

For example, the degradation process may be carried out at pH 5 to pH 11, preferably at pH 6 to pH 9.

In the present disclosure, in a specific embodiment, the polyester-comprising article may be pretreated prior to being brought into contact with the modified polypeptide of the present disclosure to physically or chemically change its structure, thereby increasing the contact area with the modified polypeptide.

Specifically, MHET, TPA, and/or EG resulting from the degradation by the contact may be recovered, sequentially or continuously.

Specifically, the recovered MHET, TPA, and/or EG may be further purified using all suitable purification methods and may be produced in a re-polymerizable form. More preferably, the purification may comprise a stripping process, separation by an aqueous solution, steam selective condensation, filtration and concentration of the medium after the bioprocess, separation, distillation, vacuum evaporation, extraction, electrodialysis, adsorption, ion exchange, precipitation, crystallization, concentration and acid addition dehydration and precipitation, nanofiltration, acid catalyst treatment, semi continuous mode distillation or continuous mode distillation, solvent extraction, evaporative concentration, evaporative crystallization, liquid/liquid extraction, hydrogenation, an azeotropic distillation process, adsorption, column chromatography, simple vacuum distillation and microfiltration, but is not limited thereto.

The final products MHET, TPA, and/or EG obtained by using the composition of the present disclosure may be recycled in the polymerization of polyesters.

Specifically, the obtained final products, MHET, TPA, and/or EG may be recycled as repolymerizable monomers and/or oligomers to synthesize polyester. Specifically, polyesters with the same properties may be repolymerized and mixed with another monomer and/or oligomer to synthesize, for example, new copolymers.

The polyester may be selected from polyethylene terephthalate (PET), polyethylene terephthalate glycol (PETG), polyethylene co-isosorbide-terephthalate (PEIT), polytrimethylene terephthalate (PTT), polybutylene adipate terephthalate (PBAT), polycyclohexylenedimethylene terephthalate (PCT), and polybutylene terephthalate (PBT). Specifically, the polyester may be PET.

The method of synthesizing the polyester using MHET, TPA, and/or EG is known in the art.

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1: Finding of Novel CaPETase

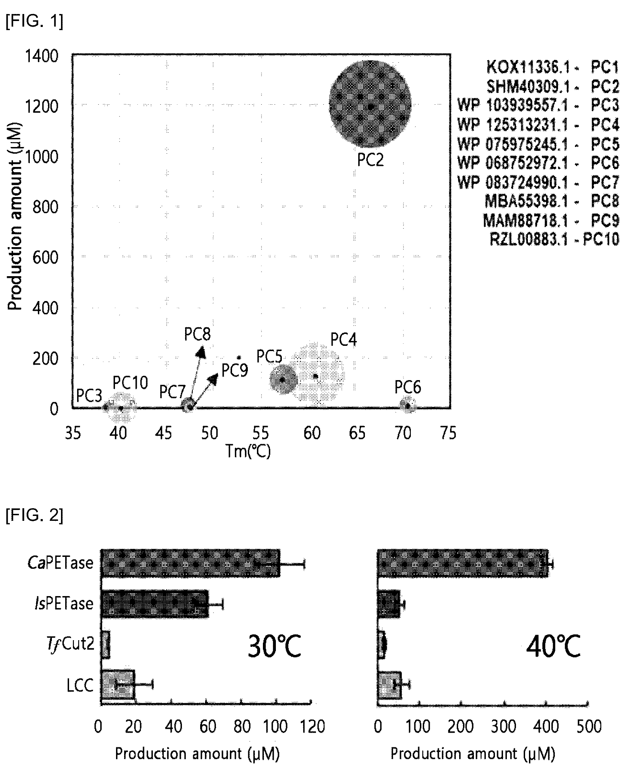

In order to find a novel PETase, in addition to the well-known PET hydrolase (IsPETase) of *Ideonella sakaiensis* 201-F6, sequence homology analysis was performed using NCBI database, resulting in 10 PETase candidates. To investigate the 10 PETase candidates thus selected, it was tried that these enzymes were first produced in a signal peptide-cleaved form, and 9 PETase candidates were successfully produced. Then, the PET hydrolyzing activities of the 9 PETase candidates were measured by monitoring the amount of released PET hydrolysates of MHET and TPA using PET bottle powder (B-PET) as a substrate. Surprisingly, most of the PETase candidates showed very small amounts of PET hydrolysates, whereas SHM40309.1 (PC2 in FIG. 1) showed a significant amount of PET hydrolysates, as compared to the other enzymes. In addition, the melting temperature (Tm) of 9 PETase candidates was measured to investigate the temperature stability of these enzymes, and these PETase candidates showed various Tm values from 38.6°C to 70.5°C. Surprisingly, SHM40309.1, which had extremely high PET hydrolyzing activity, as compared to other enzymes, also showed high temperature stability with a Tm value of 66.8°C, and SHM40309.1 showed the highest soluble expression level, as compared to other enzymes (FIG. 1).

These results indicate that SHM40309.1 has excellent characteristics for efficient PET degradation, such as enzymatic activity, temperature stability, protein expression level, etc., and therefore, SHM40309.1 (PETase of *Cryptosporangium aurantiacum,* CaPETase) was selected as a novel PETase in the present disclosure.

### Example 2: Preparation of CaPETase and Known PET Degradation Proteins

Expression and purification were performed under the following conditions. A gene codon-optimized for *E. coli* was synthesized, and amplified by polymerase chain reaction (PCR). A nucleotide sequence corresponding to the signal peptide was removed from the synthesized DNA. The PCR product was then subcloned (Ncol and Xhol) into pET22b(+) (Novagen) which lacks its own signal peptide. The resulting expression vector pET22b(+): CaPETase was used to transform *E. coli* BL21(DE3)-T1R strain. The *E. coli* strain was cultured in a flask comprising 1 L of a lysogeny broth medium supplemented with 200 mg/L ampicillin at 37°C to an optical density at 600 nm of 0.6.

Protein expression was induced by adding 0.1 mM isopropyl β-D-1-thiogalactopyranoside (IPTG), and the culture medium was further cultured at 18°C for 16 hours. Subsequently, the cells were harvested by centrifugation at 4000 ×g for 20 minutes at 4°C.

The cell pellet was resuspended in buffer A (50 mM Na₂HPO₄-HCl, pH 7.0) and disrupted by sonication. Cell debris was removed by centrifugation at 13500 ×g for 25 minutes, and the supernatant was applied to a Ni-NTA agarose column (Qiagen). After washing with buffer A comprising 30 mM imidazole, the bound protein was eluted with 300 mM imidazole in buffer A. Finally, trace contaminants were removed by size-exclusion chromatography using a Superdex 200 prepgrade column (320 ml, GE Healthcare) equilibrated with buffer A. All purification steps were performed at 4°C. The purity of the protein was examined by sodium dodecyl sulfate polyacrylamide gel electrophoresis. The purified protein was concentrated in 50 mM Na₂HPO₄-HCl (pH 7.0) and 100 mM NaCl. In the same manner, already widely known PET hydrolases, LCC (GenBank: AEV21261.1), IsPETase (GenBank: GAP38373.1) and TfCut2 (Uniprot Accession E5BBQ3), were prepared and used as comparative groups. The primers used for cloning are listed in Table 1 below.

**[Table 1]**

| SEQ ID NO. | Enzyme | Primer | Sequence |
|---|---|---|---|
| SEQ ID NO: 6 | CaPETase ^{WT} | F | TATATCATATGGCGGCCGACAACCCCTACCAAC |
| SEQ ID NO: 7 | | R | TATATCTCGAGGAACGGGCAGGTGTTCATCGAC |
| SEQ ID NO: 8 | IsPETase^{W T} | F | TATACATATGCGCGGTCCGAA TCCGACAGCC GCC |
| SEQ ID NO: 9 | | R | GCGCCTCGAGGCTGCAATTCGCTGTACGAAAATC |
| SEQ ID NO: 10 | TfCut2^{WT} | F | GCGCCATATGGCCAACCCCTACGAACGTGG |
| SEQ ID NO: 11 | | R | GCGCCTCGAGGAATGGGCAGGTCGAGC |
| SEQ ID NO: 12 | LCC^{WT} | F | TATATCATATGCAATCCAACCCGTACCAG CGCGG |
| SEQ ID NO: 13 | | R | TATATCTCGAGCTGGCAGTGGCGGTTGTTCGTC |

### Example 3: Analysis of PET Degradation Activity of CaPETase and Known PET Degradation Proteins

To compare the PET hydrolyzing activity of CaPETase with those of 3 types of PET hydrolases (LCC, IsPETase and TfCut2) prepared in Example 2, 15 mg of B-PET was prepared and immersed in 1 mL of 50 mM glycine-NaOH (pH 9.0) buffer, together with 500 nM enzyme. B-PET (PET sample derived from PET bottles) was obtained through the following processing. Transparent PET bottles were crushed using a crusher, and then the crushed PET was melted in a high-temperature oven at 270°C. The molten PET was immediately immersed in 4°C water to solidify. The quenched PET thus obtained went through a cryogenic grinding process, and PET powder of 300 µm or less was obtained through a steel mesh. The reaction mixture was reacted at 30°C, 40°C for 12 hours. Then, the reaction mixture was analyzed using HPLC. After the reaction, CaPETase completely converted the PET powder into MHET and TPA, and the products were analyzed through HPLC to evaluate the PET degradation activity.

In the reaction at 30°C, CaPETase showed extremely high PET degradation activity, as compared to LCC and TfCut2, and was similar to or even higher than IsPETase, which is known to have the highest activity at ambient temperature among PET degradation proteins reported until now. In particular, CaPETase showed 1.6 times higher activity than IsPETase for B-PET, which is a highly crystalline PET sample. The difference in the PET hydrolyzing activity of these two PET hydrolases was much greater than that of other PET hydrolases reacted at 40°C, and the activity of CaPETase was 7.5 times higher than that of IsPETase for B-PET. These results imply that CaPETase may have higher temperature controllability and PET hydrolyzing activity than IsPETase (FIG. 2).

### Example 4: Structural Analysis of CaPETase

To provide a structural basis for the high PET-hydrolyzing performance of CaPETase, the crystal structure of CaPETase was determined at a resolution of 1.36 Å. Crystallization was performed at 20°C by a sitting-drop vapor diffusion method (Fowlis, William W., et al. "Experimental and theoretical analysis of the rate of solvent equilibration in the hanging drop method of protein crystal growth." Journal of Crystal Growth 90.1-3 (1988): 117-129.) using a crystal screening kit: Index and PEG/lon (Hampton Research) and Wizard I and II (Rigaku). The experiment consisted of 1.0 µl of a protein solution and 1.0 µl of a reservoir solution, which were then equilibrated with 50 µl of the reservoir solution. The crystal was transferred to a cryoprotectant solution comprising 25%(v/v) glycerol, and extracted using a loop larger than the crystal, and immersed in liquid nitrogen, and rapidly frozen. Data were collected at 100 K via Beamline 7A from the Pohang Accelerator Laboratory (Pohang, Korea) for analysis data collection of the protein crystal. The data were indexed and integrated and scaled using the HKL2000 software suite. As a result, the CaPETase crystal belongs to a space group P2₁2₁2, and has unit cell parameters a = 82.31 Å, b = 82.45 Å, c = 87.39 Å, α = β = γ = 90° (Aroyo, Mois Ilia. International tables for crystallography. John Wiley and Sons Limited, 2013). For one molecule of CaPETase on an asymmetric unit basis, the Metthews modulus was 2.68 Å³/Da, which corresponds to a solvent content of 52.25%. In order to investigate the structural characteristics of the protein crystal, *Thermobifida cellulosilytica* (PDB code 5LUI)-derived cutinase 1 structure was employed as a search model to identify the structure of CaPETase via a molecular substitution method using the CCP4 version of MOLREP. A model building was performed using WinCoot program, and purification was carried out using REFMAC5. The statistical data are shown in Table 2. The refined model of CaPETase is stored in Protein Data Bank as PDB code 7YM9.

**[Table 2]**

| | *Ca*PETase |
|---|---|
| PDB code | 7YM9 |

| Data collection | |
|---|---|
| Wave length (Å) | 0.97934 |
| Unit cell (a, b, c; α, β, γ) (Å;°) | 82.309, 82.309, 87.391; 90.0, 90.0, 90.0 |
| Space group | P2₁2₁2₁ |
| Solvent content (%) | 52.25 |
| Protein chains in AU | 2 |
| Resolution range (Å) | 26.23-1.34 |
| Highest resolution shell (Å) | 1.36-1.34 |
| Unique reflections | 133696 |
| Redundancy | 12.4(10.4) |
| Completeness (%) | 99.7(99.8) |
| R_{merge} (%) | 8.0(51.2) |
| Average I/σ (I) | 52.7(4.4) |

| Refinement | |
|---|---|
| R(%) | 17.2 |
| R_{free}(%) | 18.3 |
| Mean B value (Å²) | 13.8 |
| RMS deviation bond lengths (Å) | 0.017 |
| RMS deviation bond angles (°) | 2.001 |
| Number of amino acid residues | 514 |
| Number of water molecules | 470 |

| Ramachandran plot | |
|---|---|
| Most favored regions (%) | 95.10 |
| Additional allowed Regions (%) | 0.41 |

### Example 5: Preparation of CaPETase Variants and Measurement of Thermal Stability and PET Degradation Activity of Variants

Based on the structure analyzed in Example 4, it was intended to improve the PET degradation activity and thermal stability of the enzyme. Two types of CaPETase variants into which mutations were introduced through the structural analysis of Example 4 were prepared in the same manner as Example 2, and the PET degradation activity was evaluated in the same manner as Example 3.
M1: CaPETaseN109A
M2: CaPETaseA155R
M3: CaPETaseN109A/A155R

The thermal stability was examined by measuring the melting temperature (the temperature at which protein denaturation occurs, Tm) of each variant. The melting temperature was determined by measuring a melting curve by StepOnePlus Real-Time PCR (Thermo Fisher Scientific) using a protein thermal shift dye (Applied Biosystems). In detail, 5 µg of CaPETase was mixed with 20 µl of a protein thermal shift dye, and signal changes reflecting protein denaturation was monitored while changing the temperature by 1 degree increments from 20°C to 90°C. Based on the melting curve, the melting temperature (Tm) of CaPETaseWT and two types of variants was determined.

M1 variant showed a Tm value of 3.2°C higher than that of CaPETaseWT, and its PET degradation activity increased by about 1.06 times. M2 variant showed a Tm value of 0.4°C higher than that of CaPETaseWT, and its PET degradation activity increased by about 1.21 times (Table 3). Next, a combination of the two mutations identified above was introduced to prepare M3 variant, and the PET degradation activity and Tm value of M3 variant were examined. As a result, as compared to CaPETaseWT, its Tm value increased by 2.8°C, and its PET degradation activity increased by about 1.32 times (Table 4). Based on this, it was confirmed that when two surface charge-modifying mutations were introduced into CaPETase protein, the PET degradation activity as well as the temperature stability of the protein was improved.

**[Table 3]**

| Variant | Concentration of product (uM) | Tm (°C) |
|---|---|---|
| CaPETaseWT | 712.75 | 66.8 |
| M1(CaPETaseN109A) | 756 | 70 |
| M2(CaPETaseA155R) | 862.56 | 67.2 |

**[Table 4]**

| Variant | Concentration of product (uM) | Tm (°C) |
|---|---|---|
| CaPETaseWT | 812.16 | 66.7 |
| M3(CaPETaseN109A/A155R) | 1072.05 | 69.5 |

### Example 6: Effects according to CaPETase Mutation Site

In order to examine changes in the protein thermal stability and PET degradation activity when the site of the surface charge-modifying mutations identified in Example 5 was replaced by different amino acids, the following variants were prepared in the same manner as Example 2, and the PET degradation activity and thermal stability were evaluated in the same manner as Example 4 and shown in Table 5.

**[Table 5]**

| Variant | Concentration of product (uM) | Tm (°C) | Variant | Concentration of product (uM) | Tm (°C) |
|---|---|---|---|---|---|
| CaPETaseWT | 712.75 | 66.8 | M22 (CaPETaseA155I) | 689.94 | 65.5 |
| M4 (CaPETaseN10 9I) | 719.88 | 66.2 | M23 (CaPETaseA155L) | 674.43 | 66.9 |
| M5 (CaPETaseN10 9L) | 696.36 | 65.1 | M24 (CaPETaseA155V) | 735.14 | 66.2 |
| M6 (CaPETaseN10 9V) | 753.38 | 65.8 | M25 (CaPETaseA155M ) | 681.39 | 67.2 |
| M7 (CaPETaseN10 9M) | 603.36 | 63.6 | M26 (CaPETaseA155F) | 610.83 | 63.9 |
| M8 (CaPETaseN10 9F) | 667.13 | 65.6 | M27 (CaPETaseA155Y) | 578.75 | 63.5 |
| M9 (CaPETaseN10 9Y) | 509.47 | 65.4 | M28 (CaPETaseA155W ) | 528.15 | 63.1 |
| M10 (CaPETaseN10 9W) | 497.78 | 62.3 | M29 (CaPETaseA155S) | 674.43 | 66.9 |
| M11 (CaPETaseN10 9S) | 507.19 | 65.6 | M30 (CaPETaseA155T) | 627.22 | 65.8 |
| M12 (CaPETaseN10 9T) | 562.07 | 66 | M31 (CaPETaseA155N ) | 539.32 | 65.9 |
| M13 (CaPETaseN10 9Q) | 697.00 | 67 | M32 (CaPETaseA155Q ) | 548.10 | 65.8 |
| M14 (CaPETaseN10 9C) | 554.52 | 64.3 | M33 (CaPETaseA155C ) | 528.15 | 65.5 |
| M15 (CaPETaseN10 9G) | 511.52 | 62.8 | M34 (CaPETaseA155G ) | 382.03 | 64.2 |
| M16 (CaPETaseN10 9P) | 301.14 | 61.6 | M35 (CaPETaseA155P) | 617.24 | 63.7 |
| M17 (CaPETaseN10 9R) | 690.58 | 64 | M36 (CaPETaseA155H ) | 652.17 | 64.8 |
| M18 (CaPETaseN10 9H) | 615.25 | 64 | M37 (CaPETaseA155E) | 651.45 | 65.3 |
| M19 (CaPETaseN10 9K) | 586.31 | 63.5 | M38 (CaPETaseA155D ) | 541.69 | 64 |
| M20 (CaPETaseN10 9D) | 655.73 | 65.7 | M39 (CaPETaseA155E) | 502.27 | 63.8 |
| M21 (CaPETaseN10 9E) | 612.96 | 66.3 | | | |

The above results confirmed that when the two mutation sites of CaPETase were changed into different amino acids, the substitution with some amino acids affected the improvement of protein activity or thermal stability. This means that the tested amino acid positions directly/indirectly affected the activity or thermal stability of the protein.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within the metes and bounds of the claims, or equivalents of such metes and bounds are therefore intended to be embraced by the claims.

## Claims

1. A modified polypeptide having a polyethylene terephthalate (PET) degradation activity,
wherein i) the modified polypeptide has at least 70% and less than 100% sequence identity to SEQ ID NO: 1; and/or
ii) the modified polypeptide is a polypeptide encoded by a polynucleotide having at least 70% and less than 100% sequence identity to a coding sequence of a mature polypeptide of SEQ ID NO: 1; and/or
iii) the modified polypeptide is a polypeptide encoded by a polynucleotide that hybridizes with (a) the coding sequence of the mature polypeptide of SEQ ID NO: 1, (b) cDNA thereof, or (c) the full-length complement of (a) or (b) under low stringency conditions, medium stringency conditions, medium-high stringency conditions, high stringency conditions, or very high stringency conditions; and/or
iv) the modified polypeptide is a functional fragment of the polypeptide of i), ii), or iii) that has the PET degradation activity; and
the modified polypeptide comprising any one modification selected from the following modifications:
deletion of amino acids, insertion of the amino acids, substitution with different amino acids, and/or a combination thereof at any one or more positions of positions 155 and 109;
wherein, the position number is a position corresponding to the position of the polypeptide of SEQ ID NO: 1.

2. The modified polypeptide of claim 1, wherein before modification of the modified polypeptide having the PET degradation activity, the amino acid at position 155 is alanine (A); and/or the amino acid at position 109 is asparagine (N).

3. The modified polypeptide of claim 1, wherein the modified polypeptide comprises modifications of amino acids at positions selected from the following positions:
155;
109;
155 and 109;
wherein, the position number is a position corresponding to the position of the polypeptide of SEQ ID NO: 1.

4. The modified polypeptide of claim 1, wherein the modified polypeptide comprises any one or more substitutions of the following substitutions:
a substitution of the amino acid corresponding to position 155 with arginine, leucine, methionine, valine, or serine; and
a substitution of the amino acid corresponding to position 109 with alanine, isoleucine, valine, or glutamine;
wherein, the position number is a position corresponding to the position of the polypeptide of SEQ ID NO: 1.

5. The modified polypeptide of claim 1, wherein the modified polypeptide comprises any one or more modifications selected from the following modifications:
i) a modification of alanine at position 155 with arginine, leucine, methionine, valine, or serine;
ii) a modification of asparagine at position 109 with alanine, isoleucine, valine, or glutamine; and
iii) a modification of alanine at position 155 with arginine and a modification of asparagine at position 109 with alanine:
wherein, the position number is a position corresponding to the position of the polypeptide of SEQ ID NO: 1.

6. A composition comprising the modified polypeptide of any one of claims 1 to 5.

7. The composition of claim 6, wherein the composition is used for degrading PET.

8. A polynucleotide encoding the modified polypeptide of any one of claims 1 to 5.

9. A host cell comprising the modified polypeptide of any one of claims 1 to 5; a polynucleotide encoding the modified polypeptide; a nucleic acid construct comprising the polynucleotide; and/or a vector comprising the nucleotide or the nucleic acid construct.

10. A method of preparing a modified polypeptide having a PET degradation activity, the method comprising the steps of:
culturing the host cell of claim 9; and
recovering the modified polypeptide having the PET degradation activity of any one of claims 1 to 5, which is expressed in the culturing step.

11. A method of degrading a polyester, the method comprising:
treating the polyester with the modified polypeptide of any one of claims 1 to 5; a host cell expressing the polypeptide; and/or a composition comprising the modified polypeptide.

12. The method of claim 11, wherein the polyester is PET.

13. A method of producing mono(2-hydroxymethyl) terephthalate (MHET), terephthalic acid (TPA), and/or ethylene glycol (EG), the method comprising:
bringing a polyester into contact with the modified polypeptide of any one of claims 1 to 5; a host cell expressing the polypeptide; and/or a composition comprising the polypeptide.

14. The method of claim 13, wherein the polyester is PET.

15. The method of claim 13, further comprising the step of recovering the produced MHET, TPA, and/or EG.

16. A method of producing a polyester, the method comprising the step of:
synthesizing the polyester using MHET, TPA, and/or EG produced by the method of any one of claims 13 to 15.

17. The method of claim 16, wherein the polyester is PET.

18. Use of the modified polypeptide of any one of claims 1 to 5; or a host cell expressing the modified polypeptide; or a composition comprising the modified polypeptide in degrading PET.

19. Use of the modified polypeptide of any one of claims 1 to 5 or a composition comprising the modified polypeptide for the reaction with a polyester in producing mono(2-hydroxymethyl) terephthalate (MHET), terephthalic acid (TPA), and/or ethylene glycol (EG).
